# FASCICULE DE BREVET EUROPEEN

(11) **EP 3 442 630 B1**
(45) Date de publication et mention de la délivrance du brevet: **29.01.2020**
(21) Numéro de dépôt: 17723445.7
(22) Date de dépôt: 13.04.2017
(51) Int. Cl.: A61M 15/00

(54) **DISPOSITIF DE DISTRIBUTION DE PRODUIT FLUIDE SYNCHRONISÉ AVEC L'INHALATION**
VORRICHTUNG ZUR INHALATIONSSYNCHRONISIERTEN AUSGABE EINES FLÜSSIGPRODUKTS
DEVICE FOR INHALATION-SYNCHRONISED DISPENSING OF A FLUID PRODUCT

(30) Priorité: 15.04.2016 FR 1653373
(43) Date de publication de la demande: 20.02.2019
(73) Titulaire: Aptar France SAS, 27110 Le Neubourg (FR)
(72) Inventeur: FABIEN, David, 29810 Plouarzel (FR)
(74) Mandataire: CAPRI
(86) Numéro de dépôt international: PCT/FR2017/050889
(87) Numéro de publication internationale: WO 2017/178764

(56) Documents cités:
- WO-A1-2004/028608
- WO-A1-2010/003846
- WO-A1-2013/178951
- WO-A2-2008/070516
- NZ-A- 562 769
- US-A- 3 456 646
- US-A- 5 119 806
- US-A1- 2008 156 321

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Les documents WO2004028608, US3456646, US5119806, NZ562769, US2008156321, WO2008070516, WO2010003846 et WO2013178951 décrivent des dispositifs de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant par rapport audit corps, une valve doseuse comportant une soupape étant assemblée sur ledit réservoir pour distribuer sélectivement le produit fluide, ledit dispositif comportant:
- un élément de blocage déplaçable et/ou déformable entre une position de blocage, dans laquelle ladite valve doseuse ne peut pas être actionnée, et une position d'actionnement, dans laquelle ladite valve doseuse peut être actionnée,
- un élément déclencheur déplaçable et/ou déformable entre une position de verrouillage, dans laquelle il bloque ledit élément de blocage dans sa position de blocage, et une position de libération, dans laquelle il ne bloque pas ledit élément de blocage, et
- un système de déclenchement commandé par l'inhalation comportant un organe sensible à l'inhalation, déformable et/ou déplaçable sous l'effet de l'inhalation, ledit organe sensible à l'inhalation coopérant avec ledit élément déclencheur, de sorte que lorsque ledit organe sensible à l'inhalation se déforme et/ou se déplace, il déplace et/ou déforme ledit élément déclencheur vers sa position de libération, permettant ainsi le déplacement et/ou la déformation dudit élément de blocage de sa position de blocage vers sa position d'actionnement, ledit élément de blocage comportant une projection qui, en position de verrouillage de l'élément déclencheur, coopère avec un épaulement de verrouillage dudit élément déclencheur pour définir une serrure empêchant le déplacement et/ou la déformation dudit élément de blocage hors de sa position de blocage, ladite serrure formant, en position de verrouillage de l'élément déclencheur, un premier point de contact entre ledit élément de blocage et ledit élément déclencheur, ledit élément de blocage comportant une projection latérale qui, en position de verrouillage de l'élément déclencheur, coopère avec une surface d'appui dudit élément déclencheur pour former, en position de verrouillage de l'élément déclencheur, un second point de contact entre ledit élément de blocage et ledit élément déclencheur.

Avantageusement, ledit élément de blocage est monté pivotant sur le corps autour d'un axe B et ledit élément déclencheur est monté pivotant sur le corps autour d'un axe C, lesdits axes B et C étant parallèles.

Avantageusement, ledit second point de contact est, en position de verrouillage de l'élément déclencheur, à une distance dudit axe C de l'élément déclencheur supérieure à la distance entre ledit axe C et ledit premier point de contact.

Avantageusement, un organe d'actionnement est assemblé sur le réservoir du côté axialement opposé à ladite valve doseuse, ledit organe d'actionnement comportant un manchon creux déplaçable axialement par rapport audit réservoir entre une position de repos et une position chargée, un ressort étant disposé entre le fond du réservoir et le bord supérieur fermé dudit manchon creux, de sorte que lorsque l'utilisateur appuie manuellement sur ledit organe d'actionnement pour le déplacer vers sa position chargée, ledit ressort est comprimé, pour transmettre une force axiale F audit réservoir.

Avantageusement, ledit organe d'actionnement comporte une patte de blocage, coopérant en position de repos avec ledit élément déclencheur pour l'empêcher de se déplacer vers sa position de libération.

Avantageusement, une poignée d'actionnement latérale est montée pivotante sur le corps entre une position de repos et une position d'utilisation dans laquelle elle déplace axialement ledit organe d'actionnement dans sa position chargée.

Avantageusement, ledit organe sensible à l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de verrouillage vers sa position de libération.

Avantageusement, ledit élément déclencheur est accessible manuellement par l'utilisateur, pour pouvoir être déplacé manuellement vers sa position de libération même en l'absence d'inhalation.

Avantageusement, ledit corps comporte une ouverture reliant l'embout buccal avec l'intérieur du corps, ladite ouverture étant fermée en début d'inhalation par un clapet, de sorte que le flux d'inhalation est initialement principalement transmis au système de déclenchement par l'inhalation.

Avantageusement, ledit clapet est ouvert lorsque ledit élément de blocage se déplace vers sa position d'actionnement.

Avantageusement, ledit dispositif comporte un compteur de doses électronique.

Avantageusement, ledit dispositif comporte des moyens d'émission de signaux pour communiquer à distance notamment des informations relatives aux actionnements du dispositif.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1a est une vue schématique en section d'un dispositif de distribution de produit fluide, selon un premier mode de réalisation avantageux, en position de repos,
- la figure 1b est une vue de détail de la figure 1a,
- la figure 2a est une vue similaire à celle de la figure 1, après appui sur le réservoir mais avant inhalation,
- la figure 2b est une vue de détail de la figure 2a,
- la figure 3a est une vue similaire à celle de la figure 2a, après inhalation mais avant actionnement,
- la figure 3b est une vue de détail de la figure 3a,
- la figure 4a est une vue similaire à celle de la figure 3a, après actionnement,
- la figure 4b est une vue de détail de la figure 4a,
- la figure 5a est une vue en perspective de l'élément de blocage,
- la figure 5b est une vue découpée similaire à celle de la figure 5a,
- la figure 6a est une vue en perspective de l'élément déclencheur,
- la figure 6b est une vue découpée similaire à celle de la figure 6a,
- la figure 7 est une vue en perspective éclatée du dispositif des figures 1 à 6,
- la figure 8 est une vue schématique en section d'un dispositif de distribution de produit fluide, selon un second mode de réalisation avantageux, en position de repos,
- la figure 9 est une vue similaire à celle de la figure 8, après actionnement, et
- la figure 10 est une vue en perspective éclatée du dispositif des figures 8 et 9.

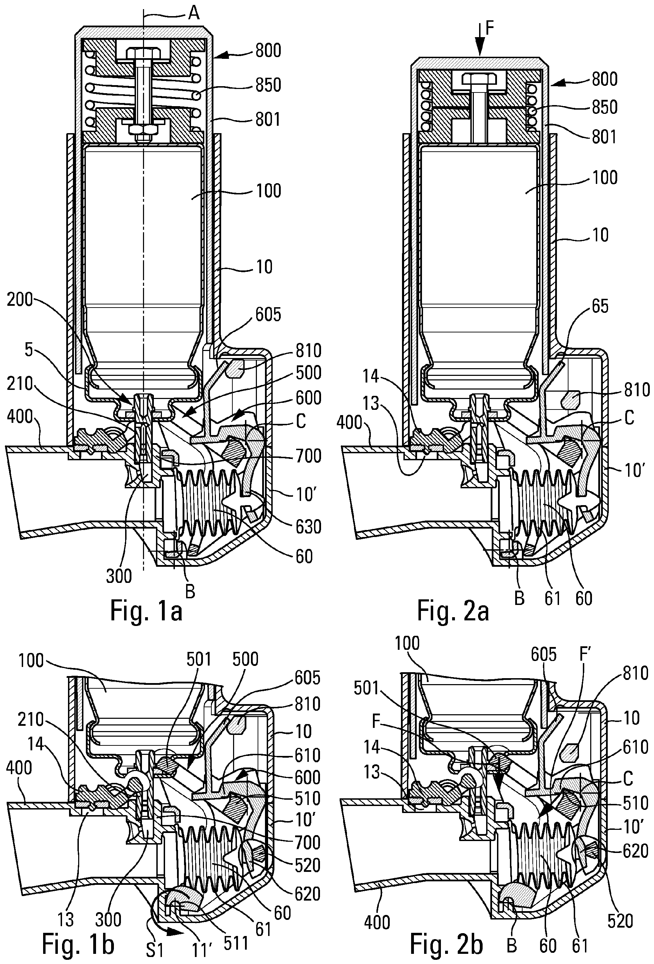

Dans la description, les termes "supérieur", "inférieur", "haut" et "bas" se réfèrent à la position du dispositif représentée notamment sur la figure 1a. Les termes "axial" et "radial" se réfèrent à l'axe central vertical A représenté notamment sur la figure 1a. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent des modes de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps principal 10 pourvu d'un embout buccal 400. Cet embout buccal 400 définit un orifice de distribution à travers lequel l'utilisateur va inhaler lors de l'utilisation du dispositif. L'embout buccal 400 peut être réalisé d'une pièce avec le corps 10, mais dans les exemples représentés sur les dessins, il est formé sur une partie inférieure de corps 10' qui se fixe audit corps principal 10. Un capot de protection amovible (non représenté) peut être prévu sur ledit embout buccal 400, notamment lors des périodes de stockage, que l'utilisateur va retirer avant utilisation.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve et une soupape 210 axialement déplaçable lors de l'actionnement par rapport audit corps de valve, et donc par rapport audit réservoir 100. Cette valve doseuse 200 peut être d'un type quelconque approprié. Elle peut être fixée au réservoir 100 par un élément de fixation, de préférence une capsule sertie 5, de préférence avec interposition d'un joint de col.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale, qui est la position d'actionnement.

L'orifice de sortie de la soupape 210 de ladite valve doseuse 200 est relié via un canal 300 audit embout buccal 400, à travers lequel l'utilisateur inhale le produit distribué. De manière connue, ladite soupape 210 est reçue dans un puits de soupape 700 qui définit au moins partiellement ledit canal 300.

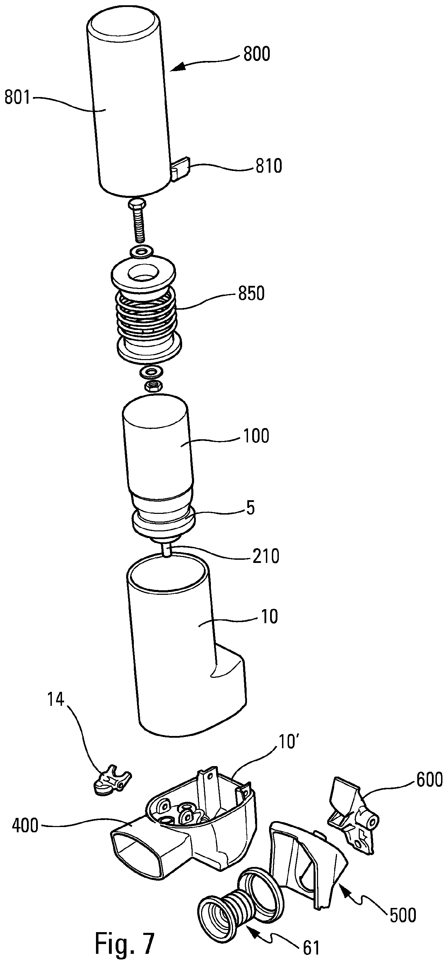

Dans l'exemple des figures 1 à 7, un organe d'actionnement 800 est assemblé sur l'extrémité supérieure du réservoir 100, opposée axialement à ladite valve doseuse 200. Cet organe d'actionnement 800 comporte un manchon creux 801 disposé dans le corps 10 autour du réservoir 100, avec un ressort 850 disposé entre le fond du réservoir 100 et le bord supérieur fermé dudit manchon creux 801. Le manchon creux 801 est déplaçable axialement par rapport audit réservoir 100 entre une position de repos et une position chargée. Ainsi, lorsque l'utilisateur souhaite déplacer le réservoir 100 axialement dans le corps 10, pour actionner la valve doseuse 200, il appuie sur ledit organe d'actionnement 800. Ceci va déplacer axialement ledit manchon creux 801 vers sa position chargée et ainsi comprimer ledit ressort 850, qui va donc transmettre une force axiale F audit réservoir 100 qui est sensiblement la même à chaque actionnement. Tant que l'utilisateur maintient son appui sur ledit organe d'actionnement 800, ledit ressort 850 est comprimé et sollicite axialement ledit réservoir 100 vers sa position d'actionnement.

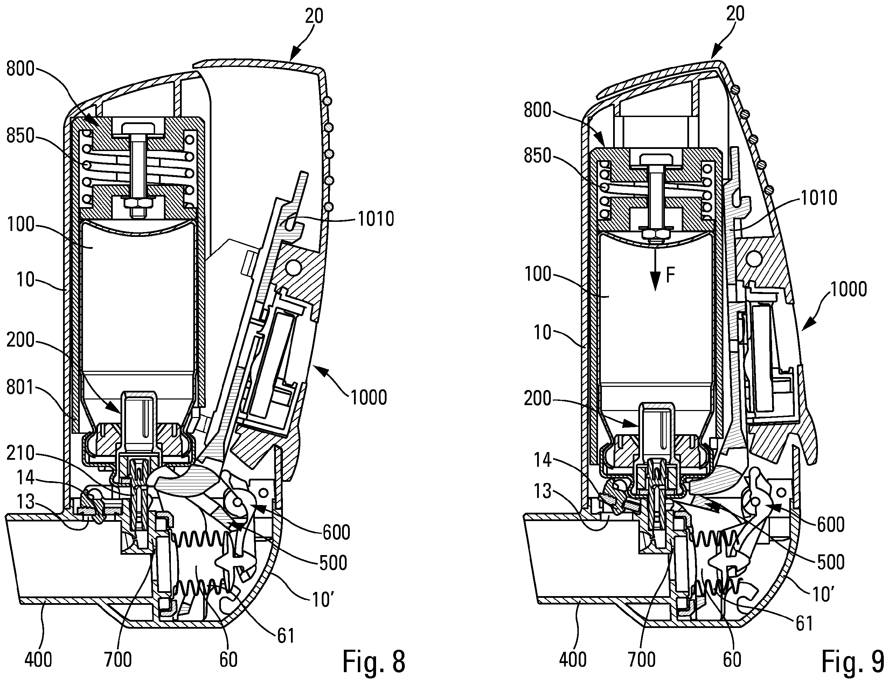

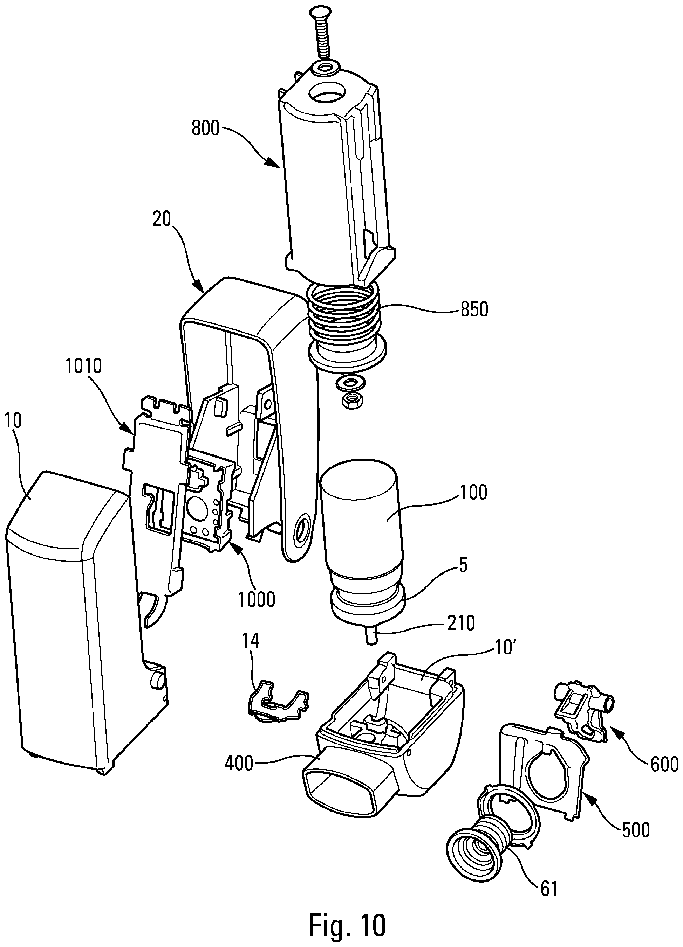

Les figures 8 à 10 illustrent une variante avantageuse, dans laquelle une poignée d'actionnement latérale 20 est montée pivotante sur le corps 10. Cette poignée, lorsque déplacée de sa position de repos, visible sur la figure 8, vers sa position d'utilisation, visible sur la figure 9, vient déplacer axialement ledit organe d'actionnement 800 pour comprimer le ressort 850. Ceci peut notamment se faire au moyen d'une came sur la poignée 20 coopérant avec un profil complémentaire sur l'organe d'actionnement 800. Tant que l'utilisateur maintient son appui sur ladite poignée, ledit ressort 850 est comprimé et sollicite axialement ledit réservoir 100 vers sa position d'actionnement. Avantageusement, ladite poignée 20 comporte un organe de sollicitation qui sollicite ladite poignée 20 vers sa position de repos. Ainsi, lorsque l'utilisateur relâche sa pression sur la poignée 20, celle-ci revient automatiquement vers sa position de repos. Ceci permet d'éviter le risque qu'après actionnement de la valve doseuse 200, cette dernière reste en position actionnée, ce qui pourrait avoir pour conséquence que la chambre de la valve se remplit d'air et que la dose suivante est incomplète voire que la valve fuit. Ceci est une des problématiques rencontrées actuellement par les dispositifs existants sur le marché.

Selon l'invention, le dispositif comporte un élément de blocage 500 déplaçable et/ou déformable entre une position de blocage, dans laquelle ladite valve doseuse 200 ne peut pas être actionnée, et une position d'actionnement, dans laquelle ladite valve doseuse 200 peut être actionnée. En position de repos, ledit élément de blocage 500 est en position de blocage, et c'est l'inhalation de l'utilisateur à travers l'embout buccal 400 qui déplace et/ou déforme ledit élément de blocage 500 vers sa position d'actionnement. En d'autres termes, tant que l'utilisateur n'a pas inhalé, l'actionnement de la valve doseuse 200 est impossible, et ce n'est que lorsqu'il inhale qu'il peut actionner ladite valve doseuse 200, avantageusement par appui manuel sur un organe d'actionnement 800 coopérant avec le fond du réservoir 100.

Comme cela sera décrit plus en détails ci-après, l'élément de blocage 500, en position de blocage, empêche le déplacement axial du réservoir 100 dans le corps 10. Lors de l'inhalation, cet élément de blocage 500 est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il appuie axialement sur le réservoir 100 pour actionner la valve 200. Comme décrit précédemment, cet appui axial sur le réservoir 100 peut se faire par l'intermédiaire de l'organe d'actionnement 800 qui comprime le ressort 850. En variante, l'utilisateur pourrait directement appuyer sur le fond du réservoir 100. Dans la variante des figures 8 à 10, c'est la poignée 20 qui va générer cet appui axial, également via l'organe d'actionnement 800 dans l'exemple représenté. Enfin, on pourrait aussi utiliser un système d'actionnement automatique, qui appliquerait cet appui axial sur le réservoir 100 indépendamment de l'utilisateur.

Le dispositif comporte un système de déclenchement commandé par l'inhalation de l'utilisateur, qui est destiné à déplacer et/ou déformer ledit élément de blocage 500 de sa position de blocage vers sa position d'actionnement, lorsque l'utilisateur inhale à travers l'embout buccal 400.

Ce système de déclenchement comporte un organe sensible à l'inhalation 60 déformable et/ou déplaçable sous l'effet de l'inhalation, cet organe sensible à l'inhalation 60 étant adapté, lorsqu'il se déforme et/ou se déplace, à permettre le déplacement et/ou la déformation dudit élément de blocage 500 de sa position de blocage vers sa position d'actionnement.

Comme cela sera décrit plus en détails ci-après, l'organe sensible à l'inhalation peut être réalisé sous la forme d'une chambre d'air déformable 60, par exemple un soufflet ou une poche déformable.

En référence aux figures 1 à 7, il est représenté un premier mode de réalisation de l'invention.

L'élément de blocage 500 est avantageusement monté pivotant autour d'un axe B sur le corps 10 ou sur la partie de corps 10' entre une position de blocage et une position d'actionnement. Dans l'exemple représenté, ledit axe B passe par un bord inférieur dudit élément de blocage 500. Il peut être formé par des projections 11' prévues sur une surface inférieure de la partie de corps 10', l'élément de blocage 500 comportant des profils complémentaires 511 adaptés à pivoter sur lesdites projections 11'. D'autres mises en œuvre sont aussi possibles.

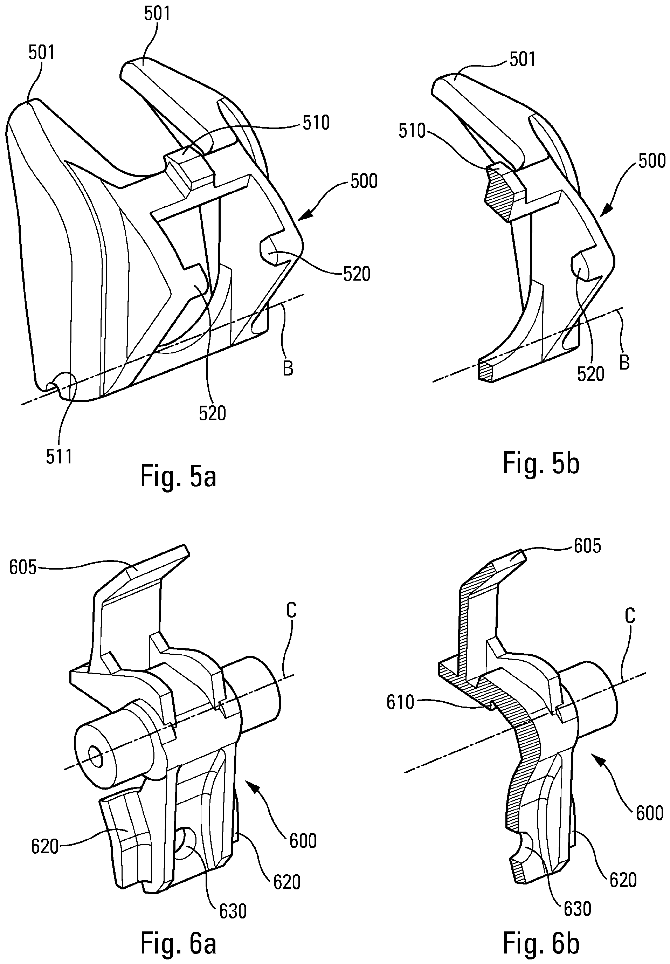

L'élément de blocage 500 comporte au moins une, de préférence deux extensions de blocage 501, qui coopèrent en position de blocage avec le réservoir 100 (avantageusement avec la capsule de sertissage 5). Les figures 5a et 5b représentent des vues en perspective et en perspective découpée de cet élément de blocage 500.

L'élément de blocage 500 est retenu en position de blocage par un élément déclencheur 600. Les figures 6a et 6b représentent des vues en perspective et en perspective découpée de cet élément déclencheur 600. Cet élément déclencheur 600 est monté pivotant autour d'un axe C sur le corps 10, sur la partie de corps 10' ou sur la poignée 20, entre une position de verrouillage, dans laquelle il bloque ledit élément de blocage 500 dans sa position de blocage, et une position de libération, dans laquelle il ne bloque plus ledit élément de blocage 500. Dans l'exemple représenté, ledit axe C passe environ au milieu dudit élément dudit élément déclencheur 600. Avantageusement, les axes B et C sont parallèles.

L'élément de blocage 500 et l'élément déclencheur 600 définissent ensemble une serrure. En particulier, ledit élément déclencheur 600 comporte un épaulement de verrouillage 610 qui, en position de verrouillage, coopère avec une projection 510 de l'élément de blocage 500, empêchant le pivotement dudit élément de blocage 500 hors de sa position de blocage. Ainsi, lorsque ledit élément déclencheur 600 est en position de verrouillage, il empêche le déplacement de l'élément de blocage vers sa position d'actionnement, ce qui bloque le déplacement axial du réservoir 100 et donc l'actionnement de la valve doseuse 200.

Cette serrure permet le déverrouillage d'un gros effort (typiquement environ 40-45N) par un petit effort généré par l'inhalation. L'élément de blocage 500 arrête la translation du bidon lorsqu'il est soumis à un effort F (de 45N par exemple) par l'appui par l'utilisateur sur l'organe d'actionnement 800. Cet élément de blocage 500 interagit avec l'élément déclencheur 600 et est bloqué/libéré par celui-ci. Le déplacement dudit élément déclencheur 600 est commandé par l'inhalation.

La géométrie de la serrure permet une amplification (effort déverrouillé/effort déverrouillant) très importante, typiquement de l'ordre de 100.

Selon l'invention, l'élément de blocage 500 et l'élément déclencheur 600 ont deux points de contact géométriquement distant:
- un premier point de contact, formé par la serrure définie entre l'épaulement de verrouillage 610 et la projection 510, se trouvant avantageusement proche de l'axe de pivotement C de l'élément déclencheur 600;
- un second point de contact éloigné du premier point de contact, formé par la coopération d'une projection latérale 520 de l'élément de blocage 500 avec une surface d'appui 620 de l'élément déclencheur 600; avantageusement, ce second point de contact est, en position de verrouillage, à une distance de l'axe C de l'élément déclencheur 600 supérieure à la distance entre ledit axe C et le premier point de contact; avantageusement, ce second point de contact est le premier contact qui se rompt lors de l'actionnement du dispositif, dès que l'utilisateur commence à inhaler.

En position de blocage, la force F générée par l'appui axial sur le réservoir 100 est appliquée sur l'élément de blocage 500 au niveau des extensions 501, avec pour effet de le faire tourner dans un sens S1, qui renforce la position fermée de la serrure et la rend stable.

La force de déverrouillage générée par l'inhalation est appliquée sur l'élément déclencheur 600 par la membrane déformable 61, de préférence en un point 630 éloigné de l'axe du pivot C; cette force de déverrouillage vise à faire tourner ledit élément déclencheur 600 dans le sens S2 contraire au sens S1.

Le couple que subit l'élément de blocage 500 est maitrisé par la distance entre l'axe d'effort sur lequel la force F est appliquée sur les extensions de blocage 501 de l'élément de blocage et l'axe de pivot B dudit élément de blocage 500. On cherche à avoir une distance la plus faible possible afin d'avoir le couple le plus faible possible.

Le couple que subit l'élément déclencheur 600 est maitrisé par la distance entre l'axe d'effort portant la force F' que subit l'élément déclencheur 600 de la part de l'élément de blocage 500 et l'axe de pivot C dudit élément déclencheur 600. Ici aussi, on cherche à avoir une distance la plus faible possible afin d'avoir le couple le plus faible possible.

La figure 2b illustre ces deux forces F et F'.

Grâce à ce système d'effort de la serrure, l'effort nécessaire pour faire pivoter l'élément déclencheur 600 est très faible et peut être généré par la membrane déformable 61, qui permet de transformer la dépression générée par l'inhalation en force de déverrouillage.

Avantageusement, la partie inférieure de corps 10' comporte une ouverture 13 reliée avec l'intérieur du corps 10. Cette ouverture 13 est fermée au repos et en début d'inhalation par un clapet 14, de sorte que le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément de blocage 500 s'est déplacé vers sa position d'actionnement sous l'effet de l'inhalation, et donc lorsque l'utilisateur peut actionner la valve doseuse 200 pour distribuer une dose de produit fluide, ledit élément de blocage 500 déplace ledit clapet 14 vers une position ouverte. Lorsque lesdites ouvertures 13 sont ainsi ouvertes, un appel d'air est généré, ce qui permet d'augmenter le flux d'inhalation. Ceci optimise la synchronisation entre la distribution de la dose et l'inhalation de l'utilisateur, et favorise une bonne distribution de la dose dans les poumons de l'utilisateur.

Avantageusement, l'élément déclencheur 600 peut être accessible de l'extérieur du corps 10 et/ou de la partie inférieure de corps 10'. Ceci permet en cas de nécessité de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité.

Dans les modes de réalisation représentés sur les figures, l'organe sensible à l'inhalation 60 est réalisé sous la forme d'une chambre d'air déformable. Avantageusement, cette chambre d'air comprend une membrane déformable 61 qui est reliée d'une part à ladite partie inférieure de corps 10' et d'autre part audit élément déclencheur 600. Avantageusement, comme visible sur les figures, la membrane 61 a une forme de soufflet et forme une chambre sensiblement étanche. D'autres formes sont possibles, notamment une simple poche ou diaphragme. Un plot peut fixer ladite membrane 61 à un orifice 630 dudit élément déclencheur 600.

Lors de l'inhalation, la membrane déformable 61 se déforme et/ou se contracte sous l'effet de la dépression générée par l'inhalation, provoquant le déplacement de l'élément déclencheur 600 de sa position de verrouillage vers sa position de libération. Ceci permet d'ouvrir la serrure définie entre l'élément de blocage 500 et l'élément déclencheur 600, et donc le déplacement dudit élément de blocage 500 de sa position de blocage vers sa position d'actionnement.

La valve 200 n'est donc actionnée qu'au moment de l'inhalation, de sorte que la dose de produit fluide est expulsée hors de l'orifice de distribution simultanément à l'inhalation.

Avantageusement, l'organe d'actionnement 800 comporte une patte de blocage 810, coopérant en position de repos avec ledit élément déclencheur 600, notamment une patte verrou 605, pour l'empêcher de se déplacer vers sa position de libération. Ainsi, si l'utilisateur inhale sans avoir exercé l'appui axial sur le réservoir 100, la serrure ne sera pas débloquée, puisque l'élément déclencheur 600 ne peut pas pivoter. La chambre d'air 60 étant sensiblement étanche, et le clapet 14 étant fermé sur l'ouverture 13, l'utilisateur se rend compte très vite qu'il ne peut inhaler correctement à travers l'embout buccal, ce qui lui rappelle qu'il faut d'abord exercer l'appui axial sur le réservoir 100 avant d'inhaler. Lorsque l'utilisateur appuie sur l'organe d'actionnement 800, il déplace axialement le manchon 801 par rapport au réservoir 100, qui lui est bloqué par l'élément de blocage 500, ce qui comprime le ressort 850. Ce déplacement axial du manchon 801 libère l'interaction entre la patte verrou 605 de l'élément déclencheur 600 et la patte de blocage 810, comme visible sur les figures 2a et 2b. Une inhalation provoquera alors un pivotement de l'élément déclencheur 600, et donc l'actionnement du dispositif, comme expliqué précédemment.

Lorsque l'utilisateur souhaite utiliser le dispositif, il place l'embout buccal 400 dans sa bouche, et exerce manuellement un appui axial sur le fond du réservoir 100, c'est-à-dire la surface supérieure dudit réservoir 100 dans la position des figures. Le réservoir 100 est bloqué et empêché de coulisser axialement dans le corps 10 par les extensions de blocage 501 de l'élément de blocage 500. Parallèlement, le blocage de l'élément déclencheur 600 est supprimé par le déplacement axial de l'organe d'actionnement 800, comme visible sur les figures 2a et 2b.

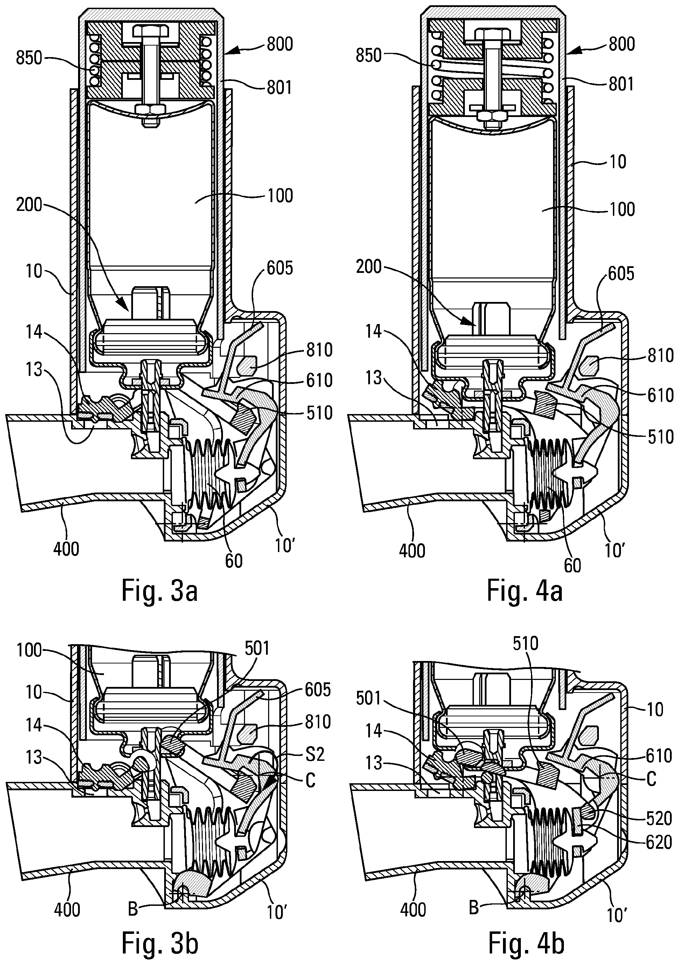

Lorsque l'utilisateur inhale à travers l'embout buccal 400, il va déformer la membrane déformable 61, ce qui va faire pivoter l'élément déclencheur 600 fixé à ladite membrane déformable 61. Ce déplacement de l'élément déclencheur 600 va libérer la serrure formée entre l'épaulement de verrouillage 610 de l'élément déclencheur 600 et la projection 510 de l'élément de blocage 500, comme visible sur les figures 3a et 3b. Sous l'effet de la force axiale transmise par le réservoir 100, générée par l'appui axial sur le fond dudit réservoir 100, l'élément de blocage 500 va pivoter permettant le coulissement axial du réservoir 100 dans le corps 10 vers sa position de distribution, et donc l'actionnement de la valve 200. Parallèlement, l'élément de blocage 500 va ouvrir le clapet 14. Cette position de distribution est représentée sur les figures 4a et 4b.

En fin d'inhalation, lorsque l'utilisateur relâche sa pression sur le fond du réservoir 100, celui-ci remonte axialement dans le corps en direction de sa position de repos sous l'effet du ressort de rappel de la valve 200, et simultanément la soupape 210 de la valve doseuse revient en position de repos, en chargeant une nouvelle dose de produit fluide dans la chambre de valve. L'élément déclencheur 600 est ramené dans sa position initiale par l'élasticité de la membrane 61 et/ou par la patte de blocage 810 de l'organe d'actionnement 800 qui revient vers sa position de repos. L'élément de blocage 500 revient dans sa position de blocage, avantageusement via à un élément élastique, tel qu'un ressort ou un élément en élastomère (non représenté).

Le dispositif est alors prêt pour une autre utilisation.

Les figures 8 à 10 illustrent une variante de réalisation comportant notamment la poignée d'actionnement latéral 20 susmentionnée et embarquant des modules électroniques.

En particulier, il est prévu un compteur de doses électronique 1000, avantageusement assemblé dans la poignée 20. Ce compteur 1000 peut notamment détecter les déplacements du réservoir 100, par exemple au moyen d'un coulisseau 1010 qui est déplacé par le réservoir 100 ou par l'élément de blocage 500, lorsqu'ils arrivent en position de distribution. En variante, le compteur 1000 pourrait être relié à un capteur, notamment un capteur à membrane, qui détecte la distribution de la dose de produit fluide, par exemple dans le puits de soupape 700. D'autres moyens d'actionner le compteur électronique 1000 sont aussi possibles, par exemple la détection du déplacement de la soupape 210 de la valve doseuse par rapport au corps de valve.

De préférence, le dispositif comporte aussi des moyens d'émission de signaux pour communiquer à distance notamment des informations relatives aux actionnements du dispositif. En particulier, le corps 10 et/ou la poignée 20 peut comporter un module d'émission de signaux, pour communiquer à distance avec une base quelconque. Des moyens d'alimentation appropriés sont avantageusement prévus.

Avantageusement, le module électronique peut comprendre notamment une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Le commutateur peut être actionné grâce au mouvement de l'élément de blocage 500, soit directement, soit via le coulisseau représenté sur les figures.

Dans l'exemple représenté, le module électronique ainsi que son coulisseau associé sont placés dans la poignée 20 mobile par rapport au corps 10. Mais en variante, on peut imaginer un module fixe par rapport au corps 10.

Associés à un compteur de dose qui compte chaque dose effectivement émise et au dispositif de synchronisation avec l'inhalation de l'invention, ces moyens d'émission de signaux permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution. Le médecin peut ainsi connaitre très exactement les conditions d'utilisation du dispositif par l'utilisateur.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à des modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10; 10') pourvu d'un embout buccal (400), un réservoir de produit (100) contenant un produit fluide et un gaz propulseur étant monté axialement coulissant par rapport audit corps (10; 10'), une valve doseuse (200) comportant une soupape (210) étant assemblée sur ledit réservoir (100) pour distribuer sélectivement le produit fluide, ledit dispositif comportant:
- un élément de blocage (500) déplaçable et/ou déformable entre une position de blocage, dans laquelle ladite valve doseuse (200) ne peut pas être actionnée, et une position d'actionnement, dans laquelle ladite valve doseuse (200) peut être actionnée,
- un élément déclencheur (600) déplaçable et/ou déformable entre une position de verrouillage, dans laquelle il bloque ledit élément de blocage (500) dans sa position de blocage, et une position de libération, dans laquelle il ne bloque pas ledit élément de blocage (500), et
- un système de déclenchement commandé par l'inhalation comportant un organe sensible à l'inhalation (60, 61), déformable et/ou déplaçable sous l'effet de l'inhalation, ledit organe sensible à l'inhalation (60, 61) coopérant avec ledit élément déclencheur (600), de sorte que lorsque ledit organe sensible à l'inhalation (60, 61) se déforme et/ou se déplace, il déplace et/ou déforme ledit élément déclencheur (600) vers sa position de libération, permettant ainsi le déplacement et/ou la déformation dudit élément de blocage (500) de sa position de blocage vers sa position d'actionnement,
ledit élément de blocage (500) comportant une projection (510) qui, en position de verrouillage de l'élément déclencheur (600), coopère avec un épaulement de verrouillage (610) dudit élément déclencheur (600) pour définir une serrure empêchant le déplacement et/ou la déformation dudit élément de blocage (500) hors de sa position de blocage, ladite serrure formant, en position de verrouillage de l'élément déclencheur (600), un premier point de contact entre ledit élément de blocage (500) et ledit élément déclencheur (600), **caractérisé en ce que** ledit élément de blocage (500) comporte une projection latérale (520) qui, en position de verrouillage de l'élément déclencheur (600), coopère avec une surface d'appui (620) dudit élément déclencheur (600) pour former, en position de verrouillage de l'élément déclencheur (600), un second point de contact entre ledit élément de blocage (500) et ledit élément déclencheur (600).

2. Dispositif selon la revendication 1, dans lequel ledit élément de blocage (500) est monté pivotant sur le corps (10; 10') autour d'un axe B et ledit élément déclencheur (600) est monté pivotant sur le corps (10; 10') autour d'un axe C, lesdits axes B et C étant parallèles.

3. Dispositif selon la revendication 2, dans lequel ledit second point de contact est, en position de verrouillage de l'élément déclencheur (600), à une distance dudit axe C de l'élément déclencheur (600) supérieure à la distance entre ledit axe C et ledit premier point de contact.

4. Dispositif selon l'une quelconque des revendications précédentes, dans lequel un organe d'actionnement (800) est assemblé sur le réservoir (100) du côté axialement opposé à ladite valve doseuse (200), ledit organe d'actionnement (800) comportant un manchon creux (801) déplaçable axialement par rapport audit réservoir (100) entre une position de repos et une position chargée, un ressort (850) étant disposé entre le fond du réservoir (100) et le bord supérieur fermé dudit manchon creux (801), de sorte que lorsque l'utilisateur appuie manuellement sur ledit organe d'actionnement (800) pour le déplacer vers sa position chargée, ledit ressort (850) est comprimé, pour transmettre une force axiale (F) audit réservoir (100).

5. Dispositif selon la revendication 4, dans lequel ledit organe d'actionnement (800) comporte une patte de blocage (810), coopérant en position de repos avec ledit élément déclencheur (600) pour l'empêcher de se déplacer vers sa position de libération.

6. Dispositif selon la revendication 4 ou 5, dans lequel une poignée d'actionnement latérale (20) est montée pivotante sur le corps (10; 10') entre une position de repos et une position d'utilisation dans laquelle elle déplace axialement ledit organe d'actionnement (800) dans sa position chargée.

7. Dispositif selon l'une quelconque des revendications précédentes, dans lequel ledit organe sensible à l'inhalation (60, 61) comporte une membrane déformable (61) définissant une chambre d'air déformable (60), ladite membrane déformable (61) étant fixée audit élément déclencheur (600), ladite membrane déformable (61) étant déformée lors de l'inhalation de telle sorte qu'elle déplace ledit élément déclencheur (600) de sa position de verrouillage vers sa position de libération.

8. Dispositif selon l'une quelconque des revendications précédentes, dans lequel ledit élément déclencheur (600) est accessible manuellement par l'utilisateur, pour pouvoir être déplacé manuellement vers sa position de libération même en l'absence d'inhalation.

9. Dispositif selon l'une quelconque des revendications précédentes, dans lequel ledit corps (10; 10') comporte une ouverture (13) reliant l'embout buccal (400) avec l'intérieur du corps (10), ladite ouverture (13) étant fermée en début d'inhalation par un clapet (14), de sorte que le flux d'inhalation est initialement principalement transmis au système de déclenchement par l'inhalation.

10. Dispositif selon la revendication 9, dans lequel ledit clapet (14) est ouvert lorsque ledit élément de blocage (500) se déplace vers sa position d'actionnement.

11. Dispositif selon l'une quelconque des revendications précédentes, comportant un compteur de doses électronique (1000).

12. Dispositif selon l'une quelconque des revendications précédentes, comportant des moyens d'émission de signaux pour communiquer à distance notamment des informations relatives aux actionnements du dispositif.

## Patentansprüche

1. Ausgabevorrichtung für ein fluides Produkt, die mit der Inhalation synchronisiert ist, umfassend einen Körper (10; 10'), der mit einem Mundansatz (400) versehen ist, einen Produktbehälter (100), der ein fluides Produkt und ein Treibgas enthält und axial gleitend in Bezug auf den Körper (10; 10') montiert ist, ein Dosierventil (200), umfassend ein federgespanntes Ventil (210), das auf dem Behälter (100) aufgesetzt ist, um das fluide Produkt selektiv auszugeben, wobei die Vorrichtung umfasst:
- ein Sperrelement (500), das zwischen einer Sperrposition, in der das Dosierventil (200) nicht betätigt werden kann, und einer Betätigungsposition, in der das Dosierventil (200) betätigt werden kann, verschiebbar und/oder verformbar ist,
- ein Auslöseelement (600), das zwischen einer Verriegelungsposition, in der es das Sperrelement (500) in seiner Sperrposition blockiert, und einer Freigabeposition, in der es das Sperrelement (500) nicht blockiert, verschiebbar und/oder verformbar ist, und
- ein Auslösesystem, das durch Inhalation gesteuert wird, umfassend ein der Inhalation gegenüber empfindliches Organ (60, 61), das unter Einwirkung der Inhalation verformbar und/oder verschiebbar ist, wobei das der Inhalation gegenüber empfindliche Organ (60, 61) mit dem Auslöseelement (600) derart zusammenwirkt, dass, wenn sich das der Inhalation gegenüber empfindliche Organ (60, 61) verformt und/oder verschiebt, dieses das Auslöseelement (600) in seine Freigabeposition verschiebt und/oder verformt, wodurch das Verschieben und/oder Verformen des Sperrelements (500) aus seiner Sperrposition in seine Betätigungsposition ermöglicht wird,
wobei das Sperrelement (500) einen Vorsprung (510) umfasst, der in der Verriegelungsposition des Auslöseelements (600) mit einem Verriegelungsabsatz (610) des Auslöseelements (600) zusammenwirkt, um eine Verschlusseinrichtung zu definieren, die das Verschieben und/oder Verformen des Sperrelements (500) aus seiner Sperrposition verhindert, wobei die Verschlusseinrichtung in der Verriegelungsposition des Auslöseelements (600) einen ersten Kontaktpunkt zwischen dem Sperrelement (500) und dem Auslöseelement (600) bildet,
**dadurch gekennzeichnet, dass** das Sperrelement (500) einen seitlichen Vorsprung (520) aufweist, der in der Verriegelungsposition des Auslöseelements (600) mit einer Auflagefläche (620) des Auslöseelements (600) zusammenwirkt, um in der Verriegelungsposition des Auslöseelements (600) einen zweiten Kontaktpunkt zwischen dem Sperrelement (500) und dem Auslöseelement (600) zu bilden.

2. Vorrichtung nach Anspruch 1, wobei das Sperrelement (500) auf dem Körper (10; 10') um eine Achse B schwenkbar montiert ist und das Auslöseelement (600) auf dem Körper (10; 10') um eine Achse C schwenkbar montiert ist, wobei die Achsen B und C parallel sind.

3. Vorrichtung nach Anspruch 2, wobei der zweite Kontaktpunkt in der Verriegelungsposition des Auslöseelements (600) in einem Abstand zur Achse C des Auslöseelements (600) liegt, der größer ist als der Abstand zwischen der Achse C und dem ersten Kontaktpunkt.

4. Vorrichtung nach einem der vorhergehenden Ansprüche, wobei ein Betätigungsorgan (800) auf dem Behälter (100) an der Seite aufgesetzt ist, die dem Dosierventil (200) axial gegenüberliegt, wobei das Betätigungsorgan (800) eine hohle Hülse (801) umfasst, die axial in Bezug auf den Behälter (100) zwischen einer Ruheposition und einer belasteten Position verschiebbar ist, wobei eine Feder (850) zwischen dem Boden des Behälters (100) und dem oberen geschlossenen Rand der hohlen Hülse (801) angeordnet ist, sodass, wenn der Benutzer manuell auf das Betätigungsorgan (800) drückt, um es in seine belastete Position zu verschieben, die Feder (850) zusammengedrückt wird, um eine axiale Kraft (F) auf den Behälter (100) zu übertragen.

5. Vorrichtung nach Anspruch 4, wobei das Betätigungsorgan (800) ein Sperrband (810) umfasst, das in der Ruheposition mit dem Auslöseelement (600) zusammenwirkt, um zu verhindern, dass es sich in seine Freigabeposition verschiebt.

6. Vorrichtung nach Anspruch 4 oder 5, wobei ein seitlicher Betätigungsgriff (20) auf dem Körper (10; 10') schwenkbar zwischen einer Ruheposition und einer Gebrauchsposition, in der er das Betätigungsorgan (800) axial in seine belastete Position verschiebt, montiert ist.

7. Vorrichtung nach einem der vorhergehenden Ansprüche, wobei das der Inhalation gegenüber empfindliche Organ (60, 61) eine verformbare Membran (61) umfasst, die eine verformbare Luftkammer (60) definiert, wobei die verformbare Membran (61) an dem Auslöseelement (600) befestigt ist, wobei die verformbare Membran (61) bei Inhalation derart verformt wird, dass sie das Auslöseelement (600) von seiner Verriegelungsposition in seine Freigabeposition verschiebt.

8. Vorrichtung nach einem der vorhergehenden Ansprüche, wobei das Auslöseelement (600) manuell durch den Benutzer zugänglich ist, um auch dann manuell in seine Freigabeposition verschoben werden zu können, wenn keine Inhalation vorliegt.

9. Vorrichtung nach einem der vorhergehenden Ansprüche, wobei der Körper (10; 10') eine Öffnung (13) umfasst, die den Mundansatz (400) mit dem Inneren des Körpers (10) verbindet, wobei die Öffnung (13) zu Beginn der Inhalation durch eine Klappe (14) verschlossen ist, sodass der Inhalationsfluss zunächst hauptsächlich auf das Auslösesystem durch Inhalation übertragen wird.

10. Vorrichtung nach Anspruch 9, wobei die Klappe (14) geöffnet wird, wenn sich das Sperrelement (500) in seine Betätigungsposition verschiebt.

11. Vorrichtung nach einem der vorhergehenden Ansprüche, umfassend einen elektronischen Dosiszähler (1000).

12. Vorrichtung nach einem der vorhergehenden Ansprüche, umfassend Signalemissionsmittel zur Fernübertragung insbesondere von Informationen betreffend die Betätigungen der Vorrichtung.

## Claims

1. An inhalation-synchronized fluid dispenser device comprising a body (10; 10') provided with a mouthpiece (400), a fluid reservoir (100) containing a fluid and a propellant gas being mounted to slide axially relative to said body (10; 10'), a metering valve (200) including a valve member (210) being assembled on said reservoir (100) for selectively dispensing the fluid, said device further comprising:
• a blocking element (500) that is movable and/or deformable between a blocking position, in which said metering valve (200) cannot be actuated, and an actuation position, in which said metering valve (200) can be actuated,
• a trigger element (600) that is movable and/or deformable between a locking position, in which it blocks said blocking element (500) in its blocking position, and a release position, in which it does not block said blocking element (500), and
• an inhalation-controlled trigger system including an inhalation-sensitive member (60, 61), deformable and/or movable under the effect of inhaling, said inhalation-sensitive member (60, 61) co-operating with said trigger element (600), so that when said inhalation-sensitive member (60, 61) deforms and/or moves, it moves and/or deforms said trigger element (600) towards its release position, thereby making it possible the moving and/or the deformation of said blocking element (500) from its blocking position towards its actuation position,
Said blocking element including (500)
a projection (510) that, in the locking position of the trigger element (600), cooperates with a locking shoulder (610) of said trigger element (600) to define a latch preventing the moving and/or deformation of said lock element (500) out of its locking position, said latch forming, in locking position of the trigger element (600), a first contact point between said blocking element (500) and said trigger element (600),
**characterized in that** said blocking element (500) includes a lateral projection (520) that, in the locking position of the trigger element (600), cooperates with a bearing surface (620) of said trigger element (600) to form, in the locking position of the trigger element (600), a second contact point between said blocking element (500) and said trigger element (600).

2. A device according to claim 1, wherein said blocking element (500) is mounted to pivot on the body (10; 10') about an axis B, and said trigger element (600) is mounted to pivot on the body (10; 10') about an axis C, said axes B and C being parallel.

3. A device according to claim 2, wherein, said second contact point is in the locking position of the trigger element (600), at a distance from said axis C of the trigger element (600) greater than the distance between said axis C and said first contact point.

4. A device according to any preceding claim, wherein an actuator member (800) is assembled on the reservoir (100) on the axially opposite side from said metering valve (200), said actuator member (800) comprising a hollow sleeve (801) that is axially movable relative to said reservoir (100) between a rest position and a primed position, a spring (850) being arranged between the bottom of the reservoir (100) and the closed top edge of said hollow sleeve (801), such that when the user presses manually on said actuator member (800) so as to move it towards its primed position, said spring (850) is compressed, so as to transmit an axial force (F) to said reservoir (100).

5. A device according to claim 4, wherein said actuator member (800) includes a blocking tab (810), cooperating in the rest position with said trigger element (600) so as to prevent it from moving towards its release position.

6. A device according to claim 4 or claim 5, wherein a laterally-actuated pusher (20) is mounted to pivot on the body (10; 10') between a rest position and a working position in which it axially moves said actuator member (800) into its primed position.

7. A device according to any preceding claim, wherein said inhalation-sensitive member (60, 61) includes a deformable membrane (61) that defines a deformable air chamber (60), said deformable membrane (61) being fastened to said trigger element (600), said deformable membrane (61) being deformed during inhaling so that it moves said trigger element (600) from its locking position towards its release position.

8. A device according to any preceding claim, wherein said trigger element (600) is accessible manually to the user, so that it can be moved manually towards its release position even in the absence of inhaling.

9. A device according to any preceding claim, wherein said body (10; 10') includes an opening (13) connecting the mouthpiece (400) to the inside of the body (10), said opening (13) being closed at the start of inhaling by a check valve (14), such that the inhalation flow due to inhaling is initially mainly transmitted to the trigger system.

10. A device according to claim 9, wherein said check valve (14) is opened when said blocking element (500) moves towards its actuation position.

11. A device according to any preceding claim, including an electronic dose counter (1000).

12. A device according to any preceding claim, including signal-transmitter means for communicating remotely in particular information relating to the actuations of the device.
